# EUROPÄISCHE PATENTANMELDUNG

(11) **EP 0 877 088 A2**
(43) Veröffentlichungstag der Anmeldung: **11.11.1998**
(21) Anmeldenummer: 98108332.2
(22) Anmeldetag: 07.05.1998
(51) Int. Cl.: C12N 15/79

(54) **Regulation der Translationsinitiation in Eukaryotenzellen**

(30) Priorität: 09.05.1997 DE 19719200
(71) Anmelder: Hoechst Research & Technology Deutschland GmbH & Co. KG, 65929 Frankfurt am Main (DE)
(72) Erfinder: Dr. Christoph Hüls, 55263 Wackernheim (DE); Dr. Karl-Christian Gallert, 61184 Karben (DE); Dr. Thomas Kiy, 65929 Frankfurt (DE); Dr. Günther Steinbrück, 72108 Rottenburg (DE); Dr. Naduparambil Korah Jacob, 72076 Tübungen (DE)

(57) **Zusammenfassung**

Die vorliegende Erfindung betrifft ein Verfahren zur Beeinflussung der Proteinexpression in eukaryotischen Zellen, wobei
- in der ersten Schleife des 3 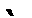 -Endes der rRNA der kleinen ribosomalen Untereinheit enthaltene
   und/oder
- dazu teilweise komplementäre in der Leadersequenzregion der mRNA enthaltende

RNA-Sequenzelemente für die Regulation der Translationsinitiation verändert und in ihrer Interaktion beeinflußt werden.

## Beschreibung

Die vorliegende Erfindung betrifft allgemein ein Verfahren zur Beeinflussung der Proteinsyntheserate in Eukaryotenzellen. Insbesondere bezieht sich das Verfahren auf die Beeinflussung der Translationsinitation in einem eukaryotischen Expressionssystem.

Bekanntlich stellt die Translation nach der Transkription den zweiten Teilprozess der Genexpression dar. Durch die Translation werden die in Form von mRNA kopierten Nukleotidzsequenzen in die Aminosäuresequenzen der Proteine übersetzt. Allgemein wird die Translation in vier Phasen unterteilt:
1. die Aminosäureaktvierung; 2. die Einleitungsphase oder Initiation; 3. die Verlängerungsphase oder Elongation; 4. die Abschlußphase oder Termination. Prinzipiell sind alle Phasen zur Beeinflussung der Translation geeignet.

Unter der katalytischen Wirkung mehrerer spezieller Proteine (Initiationsfaktoren) vereinigen sich während der Initiationsphase Initiator-tRNA, mRNA und die kleine ribosomale Untereinheit (40S bei Eukaryoten, 30S bei Prokaryoten) zu einem Initiationskomplex.

Die Bindung von mRNA an tRNA erfolgt an dem meist in der Nähe des 5'-Endes liegenden Initiationscodon, das fast immer die Sequenz AUG besitzt. Für die Bindung der mRNA an die kleine Ribosomenuntereinheit gibt es bei Prokaryoten und Eukaryoten Unterschiede. Einer dieser Unterschiede besteht darin, daß für die korrekte Initation der Translation bei Prokaryoten eine Basenpaarung zwischen dem 3'-Ende der 16S-rRNA und der Shine-Dalgarno-Sequenz auf der mRNA erforderlich ist. Die Shine-Dalgarno-Sequenz ist eine purinreiche Sequenz (3-9 Purin-Nukleotide), deren Mitte etwa 8 bis 13 Nukleotide entfernt vom 5'-Ende des AUG-Startcodons liegt.

Bei Eukaryoten besteht eine wichtige Voraussetzung für eine effiziente Initiation in der Zugänglichkeit der 5'-terminalen Cap-Struktur der jeweiligen mRNA, d.h. das Maß, mit dem die Sekundärstruktur der Leadersequenz der mRNA (5'- nichttranslatierte Region oder 5'UTR) eine Bindung der cap-bindenden Proteine erlaubt.

Diese Zugänglichkeit wird über die Sekundärstruktur der Leadersequenzregion der mRNA geregelt, welche auch für die Bindung und das Scanning (Entlangleiten) der ribosomalen 40S-Untereinheit in Richtung auf das Initiationscodon eine große Rolle spielt, in- dem sie nämlich das dem 5'-Ende der mRNA am nächsten gelegene AUG-Codon als Initiationscodon identifiziert und somit das Initiationscodon AUG von weiteren internen AUG-Codonen, welche dann für Methionin codieren unterscheidet.

Neben diesem sogenannten Ribosomen-Scanning-Prozess war bei Eukaryoten eine direkte, sequenzspezifische Wechselwirkung zwischen Ribosomen und mRNA nahezu unbekannt, wie dies bei der Shine-Dalgarno-Sequenz der Prokaryoten der Fall war. Lediglich in einem Falle wurde berichtet, daß in Säugerzellen die Leadersequenzregion am 5'-Ende einer ein Schwerketten-Bindeprotein (BiP) codierenden mRNA mit Ribosomen einen internen Bindungsmeschanismus eingeht, welcher bei der korrekten Initiation der Translation eine Rolle spielt (Macejak, D.G., P.Sarnow (1991); Internal initiation of translation by the 5'-leader of a cellular mRNA).

Der Erfindung lag daher die Aufgabe zugrunde, ein Verfahren zur Verfügung zu stellen sowie Strukturen und Sequenzmotive zu finden, mit welchen die Effizienz der Initiation der Translation in eukaryotischen Expressionssystemen und damit die Syntheserate definierter Proteine gezielt beeinflußt werden können.

Die Aufgabe wurde verfahrensmäßig mit dem kennzeichnenden Merkmal des Anspruchs 1 gelöst. Vorteilhafte Ausführungsformen und Anwendungen sind Gegenstand der Unteransprüche bzw. Verwendungsansprüche.

Ausgangspunkt der Erfindung war die bekannte Tatsache, daß die Sekundärstruktur der ribosomalen RNA-Moleküle in der Evolution von Prokaryoten und Eukaryoten stark konserviert ist. Die Stabilität dieser Sekundärstrukturen beruht im wesentlichen auf den durch Watson-Crick-Basenpaarungen gebildeten Doppelhelixregionen, die durch Faltung der Moleküle gebildet werden. Die Bedeutung, welche die Stabilität dieser Helixregionen für die Funktion der Moleküle hat, läßt sich daran erkennen, daß in den Helixbereichen häufig kompensierende Mutationen zu finden sind. Dies bedeutet, daß bei Auftreten einer Mutation, welche eine Helix destabilisiert, diese Destabilisierung durch einen zweiten, kompensierenden Basenaustausch an der potentiellen komplementären Stelle behoben wird. Die Selektion sorgt so dafür, daß die Stabilität der Moleküle auf längere Sicht nicht verringert wird und die daraufberuhende Funktion optimal erhalten bleibt.

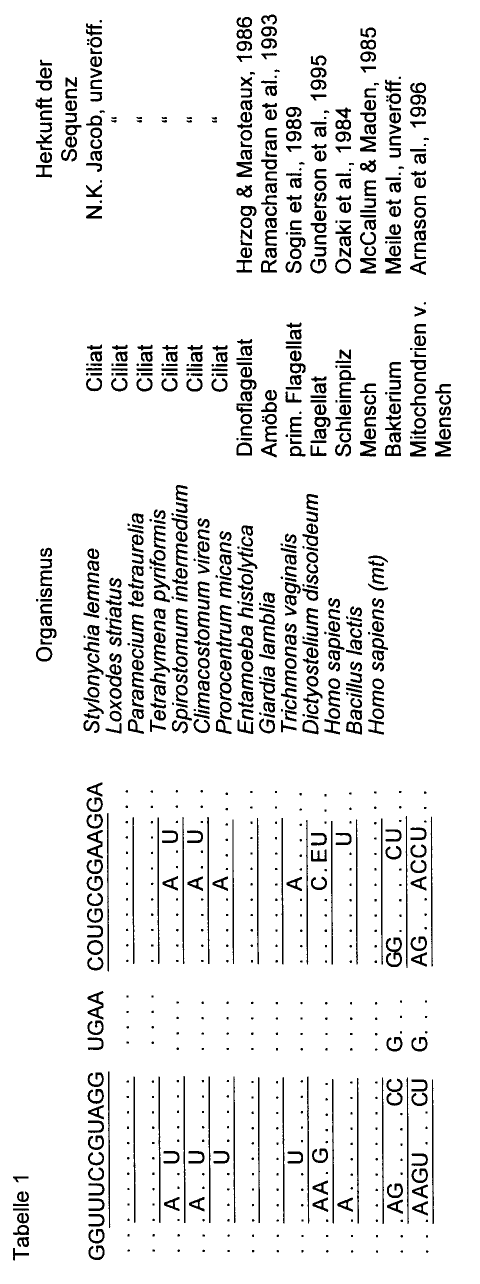

Abbildung 1 zeigt Beispiele für kompensierende Mutationen in der Helix 50 (nach dem Modell von Neefs, J.-M.,Y. Van de Peer, P. De Rijk, S. Chapelle, R. De Wachter (1993): Compilation of small ribosomal subunit RNA structures; Nucl. Acids Res. 21 (13), 4171-4184) in der rRNA der kleinen Ribosomenuntereinheit. Der kurze nicht helikale Sequenzabschnitt zwischen den gepaarten Sequenzen der Helix 50 (in unmittelbarer Nähe zum 3'-Ende der 16S-rRNA gelegen) weist eine erhebliche Sequenzkonserviertheit auf. Die darin enthaltene Vierersequenz UGAA ist bei allen in Abbildung 1 angegebenen Ciliatenarten sowie bei vielen nicht näher verwandten Eukaryotenarten einschließlich des Menschen zu finden. Wie aus den beiden untersten Zeilen der Abbildung 1 ersichtlich, ist selbst in der rRNA von Bakterien und Mitochondrien der entsprechende Sequenzabschnitt ähnlich, die hochkonservierte Vierersequenz jedoch durch einen Basenaustausch von der entsprechenden Sequenz der Eukaryoten verschieden.

Da die konservierte Schleife am äußersten Ende des 3'-Endes des rRNA-Moleküls liegt, war davon auszugehen, daß ihre Bedeutung nicht allein in der Konformation (z.B. für die Bindung ribosomaler Proteine) liegt. Die Tatsache, daß diese Sequenzelemente eine wichtige Funktion einnehmen, ist aus einem Vergleich mit den entsprechenden Leadersequenzregionen von eukayotischer mRNA zu ersehen. In Abbildung 2 sind 5'-Leader-Sequenzen der von drei Ciliatengenen transskribierten mRNAs wiedergegeben. Das Startcodon AUG ist kursiv gedruckt. Die zur Vierersequenz UGAA am 3'-Ende der ribosomalen RNA der kleinen Ribosomenuntereinheit komplementäre UUCA-Sequenz ist unterstrichen. In den 5'-Leader-sequenzen der mRNAs für ''-2-Tubulin und 8-1-Tubulin des hypotrichen Ciliaten Stylonychia lemnae und der mRNA für Calmodulin des hymenostomen Ciliaten Tetrahymena pyriformis findet man diese zur hoch konservierten Vierersequenz UGAA der kleinen Ribosomenuntereinheit komplementäre Sequenz UUCA in einem Abstand von zwei bis siebzehn Nukleotiden in 5'-Richtung vom AUG-Startcodon der jeweiligen mRNAs. Es läßt sich zeigen, daß die beschriebene Schleifenstruktur in der ribosomalen RNA über Basenpaarung mit Leaderregionen von mRNA-Molekülen eine Rolle bei der Initiation der Translation spielt. Dies ist konform mit der Tatsache, daß die Einführung von Sekundärstrukturlementen in die 5'-Leader-region von mRNAs die Translation hemmt (Sagliocco, F.A., M.R. Vega Laso, D. Zhu, M.F. Tuite, J.E.G. McCarthy, A.J. Brown (1993): The influence of 5'secondary strucutres upon ribosome binding to mRNA during translation in yeast, J.Biol.Chem. 268(35), 26522-26530). Das Ausma8 solcher Inhibition ist dabei abhängig von der Stabilität der Strukturen.

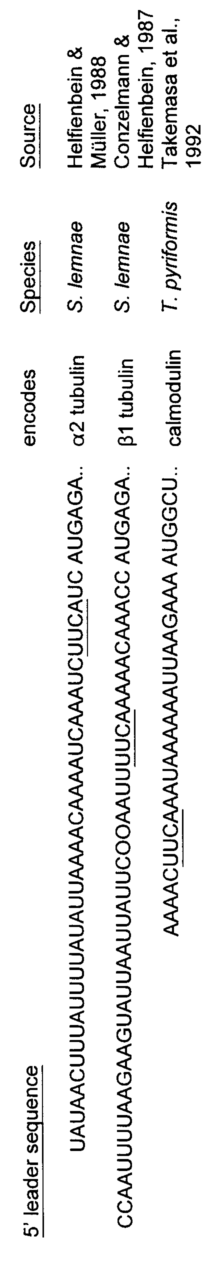

Auffällig ist, daß die Lage, Komplementarität und evulotionäre Stabilität dieses Sequenzelements stark den entsprechenden Eigenschaften der Shine-Dalgarno-Sequenz bei Prokaryonten ähnelt.

Grundlage der Erfindung ist die Erkenntnis, daß über unterschiedliche Basensequenzen in Abschnitten der Leadersequenzregion von mRNA unterschiedliche Komplementarität und damit Affinität der Leadersequenzregion zu dem für die Bindung der mRNA an die kleine Ribosomenuntereinheit zuständigen Sequenzelement in der Schleife am 3'-Ende der rRNA erzielt werden kann. In Folge davon kann man die Initiationsrate und damit auch die Proteinbiosyntheserate über die geeignete Wahl der Sequenz im fraglichen Abschnitt der Leader-Sequenz beeinflussen.

Die andere Möglichkeit für eine gezielte Beeinflussung der Initiationseffizienz der Translation auf Grundlage der oben beschriebenen Sequenzelemente besteht darin, die Sequenz der rRNA in der ersten Schleife am 3'-Ende der 16 S-RNA gezielt zu verändern, und damit auch die Komplementarität und Affinität für eine Bindung mit der Leader-Sequenz der mRNA.

Das für die Änderung der Basensequenz in Frage kommende Sequenzelement ist um die obigen hochkonservierten Vierersequenzen UGAA ( auf rRNA) und UUCA (in der Leader-Sequenz der mRNA) angesiedelt und kann sich jeweils über 8 bis 15 Nukleotide in 5'- als auch in 3'-Richtung erstrecken (im Falle der mRNA in 3'-Richtung nur dann, wenn das Startcodon AUG genügend weit von der Vierersequenz UUCA entfernt ist). Ein erfindungsgemäßes Sequenzelement kann dann 4 (hochkonservierte Vierersequenzen allein) bis 34 (Vierersequenz jeweils um 15 Nukleotide in 5'- und 3'- Richtung erweitert) umfassen, weist jedoch immer die hochkonservierte Viererfrequenz auf.

Eine Veränderung der Basensequenz in den jeweiligen Sequenzelementen kann sowohl an den Basen au8erhalb des jeweiligen hochkonservierten Vierersequenzbereichs als auch innnerhalb desselben erfolgen, sowohl einzeln als auch zusammen.

Die eingentliche, gezielte Veränderung der Basensequenz für die jeweiligen regulatorischen RNA-Sequenzelemente erfolgt auf DNA-Ebene an den jeweiligen Gensequenzen nach an sich bekannten Verfahren für oligonukleotidgerichtete in vitro Mutagenese.

Damit die entsprechenden Transkriptionsprodukte rRNA bzw. mRNA in einer eukaryotischen Zelle ihre gewünschte Funktion für die Translationsinitiation ausüben können, müssen die entsprechend gezielt "mutierten" Gensequenzen mittels gentechnischer Methoden in eine eukaryotische Zellen eingeschleust werden.

Von den hierfür in Frage kommenden Klonierungsstrategieen sind die einzelnen Schritte im Stand der Technik bekannt. Die Fragmentierung der die jeweilige rRNA- oder mRNA-Sequenzelemente kodierenden DNA sowie der damit eventuell einzuführenden Gene kann mittels eines gezielten Restriktionsverdaus, mit einer cDNA-Synthese an einer geeigneten mRNA-Matrize oder mittels direkter chemischer Synthese (im Falle relativ kurzer Sequenzelemente oder bei erforderlicher hoher in vitro Mutagenese) erfolgen.

Das Verknüpfen von DNA und Vektor über die Ligation von glatten oder kohäsiven Enden, den Einsatz von Linkermolekülen sowie das Ankoppeln von Homopolymerschwänzen sind Stand der Technik.

Die Wahl eines geeigneten Wirt-Vektor-Systems richtet sich insbesondere nach dem Zweck, der mit der Klonierung verfolgt werden soll.

Als eukaryotische Wirtszellen eignen sich prinzipiell alle im Stand der Technik hierfür bekannten pflanzlichen und tierischen Zellen sowie eukaryotische Mikroorganismen wie Pilze, Protozoen und Mikroalgen. In der vorliegenden Erfindung wurden bevorzugt Ciliaten eingesetzt.

Ciliaten sind einzellige, tierische Eukaryoten, die ähnlich wie Prokaryoten kultiviert werden können, womit man dann ausgehend von einzelnen Zellen durch vegatative Vermehrung identische Klone mit relativ kurzen Generationszeiten heranziehen kann. Dabei sind bei einigen Arten sehr hohe Zelldichten in kontinuierlicher oder Batch-Kultur erzielbar. In fast allen Ciliatenzellen findet man zwei unterschiedlich differenzierte Typen von Zellkernen: kleine transkriptionsinaktive meist diploide Mikronuclei und meist sehr gro8e, DNA-reiche Makronuclei. Die Mikronuclei haben hauptsächlich generative Funktion, d.h. im Verlauf der Konjugation entstehen aus ihnen durch meiotische Teilungen haploide Gamentenkerne. Die Makronuklei steuern alle somatischen Vorgänge der Zellen, wobei ihr Genom permanent transkribiert. Darüber hinaus liegen bei fast allen Ciliaten die Gene im Makronucleus mehr oder weniger stark amplifiziert vor, was zu hohen Expressionsraten schon unter normalen Bedingungen führt. Je nach betrachtetem Gen und Ciliatenart können die Kopienzahlen bis zu mehreren Millionen betragen. Der Grad der Amplifikation mancher Gene kann durch geeignete Ma8nahmen zusätzlich noch erhöht werden.

Da die Ciliatenarten fast alle für Eukaryoten typischen Eigenschaften aufweisen insbesondere bei der DNA-Replikation, der Transkription und dem Processing, der Translation und deren Regulation, der Endo- und Exocytosevorgänge usw. sind sie aufgrund ihrer einzigartigen Genomorganisation zur Untersuchung und insbesondere Gewinnung von regulatorisch wirkenden Genomkomponenten besonders geeignet.

Als Vektoren kommen die im Stand der Technik für Pilze, Pflanzen- und Tierzellen geeigneten Vektoren in Frage. Bei Verwendung von Hefezellen als Wirt läßt sich die Vielzahl der für diese Zellen geeigneten Hefeplasmide einsetzen. Neben episomalen (YEps) und integrativen Hefeplasmiden (YIps) sind insbesondere replikative Hefeplasmide (YRps) bevorzugt, da sich diese nicht in Chromosomen festsetzen und extrachromosomal autonom replizieren. Geeignete Vektoren für Pflanzenzellen sind beispielsweise Plasmide, wie die Ti-Plasmide, oder Viren, wie das Blumenkohl-Mosaik-Virus. Geeignet für Tierzellen sind ebenfalls Viren (z.B. Baculoviren im Falle von Insekten, Papillomaviren, SV40) und Plasmide (bevorzugt Hybridvektoren mit Teilen des SV40-Genoms).

Für die in dieser Erfindung bevorzugt eingesetzten Ciliaten als Wirt kommt in einer bevorzugten Ausführungsform das Plasmid pRT103gus als Vektor zum Einsatz. Es trägt eine Ampicilinresistenz, den 35S-Promotor des Blumenkohl-Mosaik-Virus, die codierende Region des β-Glucuronidase-Gens von E.coli sowie ein Polyadenylierungssignal.

Die Wahl einer geeigneten Methode für den DNA-Transfer in die Wirtszelle richtet sich nach der Kombination von Wirtszelle und Vektor. Für das Einschleusen der DNA in die Zellen kommen sowohl biologische Systeme in Frage, wie beispielsweise ein Virus oder ein anderes infektiöses Agens als Vektor, oder man kann die klonierten Vektoren direkt in die Wirtszellen einführen. Während Tierzellen relativ zart sind und sich deshalb leicht transformieren lassen, weisen Pflanzenzellen eine starre Zellwand auf, die einem einfachen DNA-Transfer im Wege steht. Wird aber die Zellwand enzymatisch entfernt, so wird ein Protoplast erzeugt, der sich z.B. mit Hilfe einer Elektroporation transformieren läßt. Bei dieser Technik wird die Zellmembran mit einem elektrische Impuls vorübergehend durchlässig gemacht, so daß die DNA eindringen kann; anschließend regenerieren sich die Protoplasten wieder. Bei einer anderen Form der Transformation schleust man die DNA mechanisch in die Zellen ein. Mit dem Verfahren der Mikroinjektion wird die DNA mit einer äußerst feinen Nadel direkt in die Zelle bzw. den Kern injiziert.

Das in der vorliegenden Erfindung vorteilhafterweise eingesetzte Wirt-Vektor-System Ciliat-pRT103gus zeigte mit den üblichen Transformationsmethoden von Eukaryoten, wenn überhaupt, nur geringe Transformationsraten. Darüber hinaus erwiesen sich bei Ciliaten einige Selektionsmarker, die bisher im Stand der Technik erfolgreich eingesetzt wurden, als nicht anwendbar. Der Grund ist offensichtlich darin zu suchen, daß Ciliatenzellen von einer komplexen stabilen Cortexstruktur umgeben sind.

Als geeignet für einen erfolgreichen DNA-Transfer erwies sich ein seit kurzem im Stand der Technik bekanntes Verfahren, mit welchem man von einer festen und starren Zellwand umgebene Pflanzenzellen erfolgreich transformieren konnte (Sandford, J.C., F.D.Smith, J.A.Russel (1993): Methods in Enzymology 217, 483-509), das Microcarrierbombardement mittels einer sog. Genpistole. Mit dieser Methode werden Zellen mit DNA-beschichteten Microcarriern oder Mikroprojektilen beschossen. Hierfür werden die Mikroprojektile (im allgemeinen Schwermetallpartikel) mit der zu transferierenden DNA beschichtet. Sodann werden die Mikroprojektile auf ein Makroprojektil aufgetragen, welches in der Pistole mittels Schießpulver oder ein mit Treibgas beschleunigt wird und mündungsseitig auf eine durchbohrte Blende trifft. Diese stoppt das Makroprojektil und läßt die Mikroprojektile durch die Öffnung passieren, wonach sie dann auf die Zellen treffen und die DNA ins Zellinnere einschleusen.

In der vorliegenden Erfindung wurden Goldpartikel als Mikroprojektile eingesetzt. Die Transformation von Ciliaten mit dieser Methode ist in der deutschen Patentanmeldung 196 26 564.9 beschrieben.

Ist die Eukaryotenzelle erst einmal mit einem im Expressionsvektor integrierten Gen für ein erfindungsgemäßes regulatorisches Sequenzelement transformiert, so kann die transformierte Zelle vielseiten Einflüssen unterliegen.

Wird ein solches mit seiner Sequenz an einen gewünschten Grad für die Translationsinitiation und damit eine gewünschte Proteinsyntheserate angepaßtes Sequenzelementgen vor einem Strukturgen für ein definiertes Protein im Expressionsvektor transformiert, so läßt sich dieses Protein nach erhöhter induzierter Transkription unter dem Einfluß eines starken Promotors im Falle von mRNA als Transkript mit hoher Initiationsfrequenz translatieren, was erhöhte Ausbeuten zur Folge hat.

Wird das beschriebene, evtl. an eine gewünschte Proteinsyntheserate angepaßte ribosomale RNA-Sequenzelement in einen Organismus eingebracht, so kann die Syntheserate aller am Ribosom translatierten Proteine beeinflußt werden.

Andererseits lassen sich durch Transformation mehrere Strukturgene einführen, deren Expressionsprodukte Enzyme darstellen, die in einem definierten Fermentationsprozeß ein definiertes Substrat ab- oder umbauen. Über die im Stand der Technik bereits bekannte Möglichkeit des Proteindesigns lassen sich dann durch gezielte in vitro Mutagenese spezielle Sequenzen für die Strukturgene erhalten, welche letztendlich die katalytische Aktivität der jeweiligen Enzyme bestimmen. Die vorliegende Erfindung bietet dann zusätzlich noch die Möglichkeit, durch gezielte Wahl der Sequenz des dem eigentlichen Strukturgen jeweils vorgeschalteten das Sequenzelement umfassenden Leadersequenzgens, die Transkriptionsrate und damit das Konzentrationsverhältnis des jeweiligen Enzyms zu den anderen Enzymen vorzugeben. Dadurch wird es möglich, für einen definierten Fermentationsprozeß die dazu beteiligten Enzyme sowohl hinsichtlich ihrer katalytischen Aktivität als auch hinsichtlich ihres Konzentrationsverhältnisses optimal auf maximale Ausbeuten abzustimmen.

Umgekehrt kann aber auch die Proteinexpression gehemmt werden, indem mit dem Expressionsvektor DNA-Abschnitte transformiert werden, welche bei der Expression ein kurzes Transkript liefern, das im Sinne einer Antisense-RNA-Affinität zu dem in der Leadersequenz enthaltenen regulatorischen Sequenzelement aufweist und dieses blockiert, so daß damit die Bindung der mRNA an den in der Schleife am 3'-Ende der kleinen Ribosomomenuntereinheit gelegenen hochkonservierten Sequenzabschnitt UGAA verhindert wird.

Eine analoge Wirkung kann mit dem umgekehrten Verfahren erzielt werden, indem das Transkript der transformierten DNA-Abschnitte kurze Sequenzen aus der Leadersequenzregion der mRNA ergibt, welche befähigt sind, die hochkonservierte Bindungsstelle an der Schleife am 3'-Ende der ribosomalen Untereinheit zu blockieren und demgemäß vorzugsweise die Vierersequenz UUCA enthalten.

Eine selektive Hemmung der Translationsinitiation und damit der Proteinbiosynthese in eukariotischen Zellen ist prinzipiell mit jeder Verbindung zu erzielen, welche ein hohe Bindungsaffinität zu der die Vierersequenz UGAA aufweisenden ersten Schleife am 3'-Ende der kleinen ribosomalen RNA-Untereinheit oder zu einem die Vierersequenz UUCA umfassenden Leadersequenzelement in der Nähe des Startkodons AUG aufweist. Dies ist insbesondere bei der Bekämpfung pathogener eukaryotischer Mikroorganismen und Parasiten von Bedeutung, für welche den Antibiotika in der Bakterienbekämpfung analoge selektiv wirkende Agentien im Stand der Technik nicht bekannt waren.

## Patentansprüche

1. Verfahren zur Beeinflussung der Proteinexpression in eukaryotischen Zellen, dadurch gekennzeichnet, daß
- in der ersten Schleife des 3'-Endes der rRNA der kleinen ribosomalen Untereinheit enthaltene
und/oder
- dazu teilweise komplementäre in der Leadersequenzregion der mRNA enthaltende
RNA-Sequenzelemente für die Regulation der Translationsinitiation verändert und in ihrer Interaktion beeinflußt werden.

2. Verfahren nach Anspruch 1, dadurch gekennzeichnet, daß in die eukaryotische Zelle mit einem geeigneten Expressionsvektor rekombinante DNA mit einem die regulatorischen Sequenzelemente kodierenden DNA-Abschnitt transferiert wird.

3. Verfahren nach den Ansprüchen 1 und 2, dadurch gekennzeichnet, daß die Effizienz der Translationsinitation über ein jeweils spezifisch an die gewünschte Proteinsyntheserate angepaßte Basensequenz des DNA-Abschnitts eingestellt wird.

4. Verfahren nach den Ansprüchen 1 bis 3, dadurch gekennzeichnet, daß in der rekombinanten DNA ein DNA-Abschnitt enthalten ist, der ein regulatorisches rRNA-Frequenzelement mit der hochkonservierten Sequenz UGAA codiert.

5. Verfahren nach den Ansprüchen 1 bis 3, dadurch gekennzeichnet, daß in der rekombinanten DNA ein DNA-Abschnitt enthalten ist, der ein regulatorisches rRNA-Sequenzelement mit mindestens einem Basenaustausch in der hochkonservierten Sequenz UGAA codiert.

6. Verfahren nach einem der Ansprüche 1 bis 3, dadurch gekennzeichnet, daß in der rekombinanten DNA ein DNA-Abschnitt enthalten ist, der ein regulatorisches mRNA-Sequenzelement mit der hochkonservierten Sequenz UUCA codiert.

7. Verfahren nach den Ansprüchen 1 bis 3, dadurch gekennzeichnet, daß in der rekombinanten DNA ein DNA-Abschnitt enthalten ist, der ein regulatorisches mRNA-Sequenzelement mit mindestens einem Basenaustausch in der Sequenz UUCA codiert.

8. Verfahren nach den Ansprüchen 1 bis 3 und 6, dadurch gekennzeichnet, daß die rekombinante DNA zur Herstellung von rekombinanten Proteinen die Strukturgene für diese Proteine umfaßt sowie einen DNA-Abschnitt mit an hohe Ausbeuten der Expressionsprodukte angepaßter Basensquenz.

9. Verfahren nach Anspruch 8, dadurch gekennzeichnet, daß die rekombinanten Proteine Enzyme sind.

10. Verfahren nach den Ansprüchen 1 bis 4, dadurch gekennzeichnet, daß die Sequenz des DNA-Abschnitts an eine hohe Syntheserate aller an den jeweiligen Ribosomen translatierten Proteine des Wirtssystems angepaßt ist.

11. Verfahren nach den Ansprüchen 1 bis 4, dadurch gekennzeichnet, daß zur Hemmung der Translation in den Wirtszellen ein DNA-Abschnitt eingesetzt wird, welcher als Transkript eine zu den regulatorischen mRNA-Sequenzelementen komplementäre Antisense-RNA liefert.

12. Verfahren nach den Ansprüchen 1 bis 3 und 6, dadurch gekennzeichnet, daß zur Hemmung der Translationsinitiation in der Wirtszelle ein DNA-Abschnitt eingesetzt wird, der als Transkript ein kurzes, in der Leadersequenzregion der mRNA gelegenes, die Vierersequenz UUCA enthaltendes RNA-Stück liefert.

13. Verfahren nach einem der vorhergehenden Ansprüche, dadurch gekennzeichnet, daß die eukaryotischen Zellen aus transformierten Ciliatenzellen bestehen.

14. Ein zur Transformation von eukaryotischen Wirtszellen befähigter Vektor, in welchen ein DNA-Abschnitt inseriert ist, der als Transkirpt mindestens ein regulatorisches RNA-Sequenzelement von Eukaryoten liefert, das in der ersten Schleife des 3'-Endes der rRNA in der kleinen ribosomalen Untereinheit gelegen ist.

15. Ein zur Transformation in eukaryotische Wirtszellen befähigter Vektor, in welchen ein DNA-Abschnitt inseriert ist, der als Transkript mindestens ein regulatorisches RNA-Sequenzelement von Eukaryoten liefert, das in der Leadersequenzregion der mRNA gelegen ist und teilweise komplementär ist zu dem regulatorischen rRNA-Sequenzelement der ersten Schleife des 3'-Endes der rRNA in der kleinen ribosomalen Untereinheit.

16. Eine zur selektiven Hemmung der Translationsinitiation von eukaryotischen Zellen geeignete Verbindung, dadurch gekennzeichnet, daß sie eine hohe Bindungsaffinität zu der die Vierer-Sequenz UGAA umfassenden ersten Schleife am 3'-Ende der kleinen ribosomalen RNA-Untereinheit aufweist.

17. Eine zur selektiven Hemmung der Translationsinitation von eukaryotischen Zellen geeignete Verbindung, dadurch gekennzeichnet, daß sie eine hohe Bindungsaffinität zu einem die Vierer-Sequenz UUCA aufweisenden Leadersequenzelement in der Nähe des Startcodons AUG aufweist.

18. Verwendung von
- in der ersten Schleife des 3'-Endes der rRNA der kleinen ribosomalen Unterheit enthaltenen
und/oder
- dazu teilweise komplementären in der Leadersequenzregion der mRNA enthaltenen
regulatorischen RNA-Sequenzelementen zur Beeinflussung der Proteinexpression in eukaryotischen Zellen.

19. Verwendung der regulatorischen RNA-Sequenzelemente nach Anspruch 18 mit einer jeweils an eine gewünschte Proteinsyntheserate angepa8ten Änderung in der Basensequenz.

20. Verwendung von Antisense-RNA, die komplementär ist zu dem in der Leadersequenzregion der mRNA enthaltenen zur rRNA in der ersten Schleife des 3'-Endes der kleinen ribosomalen Untereinheit teilweise komplementären regulatorischen Sequenzelement zur Hemmung der Proteintranslation in eukaryotischen Zellen.

21. Verwendung eines RNA-Abschnitts, der komplementär ist zu der in der ersten Schleife des 3'-Endes der rRNA der kleinen ribosomalen Untereinheit enthaltenen rRNA zur Hemmung der Translation in eukaryotischen Zellen.

22. Verwendung eines Expressionsvektors zur Transformation eukaryotischer Zellen, dadurch gekennzeichnet, daß er rekombinante DNA mit einem DNA-Abschnitt umfa8t, welcher
- in der ersten Schleife des 3'-Endes der rRNA der kleinen ribosomalen Untereinheit enthaltene
und/oder
- dazu teilweise komplementäre in der Leadersequenzregion der mRNA enthaltene regulatorische RNA-Sequenzelemente codiert.

23. Verwendung einer Verbindung zur selektiven Hemmung der Translationsinitiation von eukaryotischen Zellen, dadurch gekennzeichnet, daß sie eine hohe Bindungsaffinität zu der die 4er-Sequenz UGAA umfassenden ersten Schleife am 3'-Ende der kleinen ribosomalen RNA-Untereinheit aufweist.

24. Verwendung einer Verbindung zur selektiven Hemmung der Translationsinitiation von eukaryotischen Zellen, dadurch gekennzeichnet, daß sie eine hohe Bindungsaffinität zu einem die 4er-Sequenz UUCA aufweisenden Leadersequenzelement in der Nähe des Startcodons AUG aufweist.
